# EUROPÄISCHE PATENTSCHRIFT

(11) **EP 4 260 896 B1**
(45) Veröffentlichungstag und Bekanntmachung des Hinweises auf die Patenterteilung: **25.02.2026**
(21) Anmeldenummer: 22168331.1
(22) Anmeldetag: 14.04.2022
(51) Int. Cl.: A61M 37/00

(54) **HANDGERÄT ZUM LOKALEN AUFSTECHEN EINER MENSCHLICHEN ODER EINER TIERISCHEN HAUT**
HANDHELD DEVICE FOR THE LOCAL PUNCTURING OF HUMAN OR ANIMAL SKIN
APPAREIL PORTATIF PERMETTANT DE PERFORER LOCALEMENT UNE PEAU HUMAINE OU ANIMALE

(43) Veröffentlichungstag der Anmeldung: 18.10.2023
(73) Patentinhaber: MT.DERM GmbH, 12307 Berlin (DE)
(72) Erfinder: SCHERKOWSKI, Dirk, 12489 Berlin (DE)
(74) Vertreter: Bittner, Thomas L.

(56) Entgegenhaltungen:
- EP-A1- 2 954 925
- DE-U1- 202006 013 148
- TW-A- 201 117 748
- US-A1- 2014 018 835
- US-A1- 2017 354 810
- US-A1- 2018 369 553

## Beschreibung

Die Erfindung betrifft ein Handgerät zum lokalen Aufstechen einer menschlichen oder einer tierischen Haut.

### Hintergrund

Solche Handgeräte werden verwendet, um eine Haut lokal aufzustechen, insbesondere zum Einbringen eines Farbstoffs in die Haut für ein Tattoo oder permanentes Makeup, aber auch zum Applizieren medizinischer oder kosmetischer Wirkstoffe auf die Haut, die mittels lokalen Aufstechens der Haut eingebracht werden. Die Handgeräte verfügen üblicherweise über eine Antriebsvorrichtung, die mit einem Gehäuse gebildet ist. In dem Gehäuse ist eine Antriebseinrichtung angeordnet, beispielsweise ein elektrischer Motor, mit dem eine drehende Antriebsbewegung bereitgestellt wird. Mit Hilfe eines Wandlungsmechanismus wird die drehende Antriebsbewegung in eine repetierende Vor- und Zurückbewegung gewandelt, die ihrerseits auf eine Hautstecheinrichtung übertragen wird, um ein oder mehrere Stechnadeln der Hautstecheinrichtung zum lokalen Aufstechen der Haut vor- und zurückzubewegen. Die Hautstecheinrichtung ist in einem Hautstechmodul aufgenommen, welches lösbar oder nicht lösbar mit dem Gehäuse der Antriebsvorrichtung verbunden ist, derart, dass die mittels der Antriebseinrichtung bereitgestellte Antriebskraft zum Vor- und Zurückbewegen der einen oder der mehreren Nadeln der Hautstecheinrichtung hierauf übertragen werden kann. Üblicherweise werden Stechnadelspitzen der einen oder der mehreren Stechnadeln beim Vor- und Zurückbewegen der Stechnadeln durch eine vordere Gehäuseöffnung des Hautstechmoduls aus- und eingefahren.

Bei der Verwendung des Handgeräts für unterschiedliche Anwendungen besteht häufig der Wunsch, das Ausmaß oder die Länge der Vor- und Zurückbewegung der Stechnadeln einstellen zu können, beispielsweise zum Verändern einer Stechtiefe in die Haut. Hierzu ist es bekannt, eine Hublänge zwischen einem vorderen und einem hinteren Endpunkt der Vor- und Zurückbewegung der Stechnadeln einzustellen.

US 2017/354810 A1 betrifft eine dermatologische Microneedling-Vorrichtung und US 2018/369553 A1 eine stiftartige Microneedling-Vorrichtung.

US 2014/018835 A1 und EP 2 954 925 A1 beschreiben Handgeräte zum lokalen Aufstechen einer menschlichen oder einer tierischen Haut.

DE 20 2006 013 148 U1 betrifft eine Vorrichtung zur Übertragung von axial gerichteten Kräften auf Nadeln einer Tatowier- oder Schminkmaschine.

TW 201 117 748 A betrifft eine Stützvorrichtung für eine Augenbrauen-Tätowiervorrichtung.

### Zusammenfassung

Aufgabe der Erfindung ist es, ein verbessertes Handgerät zum lokalen Aufstechen einer menschlichen oder einer tierischen Haut anzugeben, welches flexibel und mit geringem Aufwand an unterschiedliche Anwendungsbedingungen anpassbar ist.

Zur Lösung ist eine Antriebseinrichtung für ein Handgerät zum lokalen Aufstechen einer menschlichen oder einer tierischen Haut nach dem unabhängigen Anspruch 1 geschaffen. Ausgestaltungen sind Gegenstand von abhängigen Unteransprüchen.

Nach einem Aspekt ist ein Handgerät zum lokalen Aufstechen einer menschlichen oder einer tierischen Haut geschaffen, aufweisend: ein Handstück mit einem Handstück-Gehäuse; ein Hautstechmodul, welches mit dem Handstück verbunden ist und ein Modulgehäuse aufweist, an dem eine Modulgehäuseöffnung gebildet ist; eine Antriebvorrichtung, die zumindest teilweise in dem Handstück-Gehäuse angeordnet und eingerichtet ist, an einer handstückseitigen Verbindungsstelle entlang einer Arbeitsrichtung eine Vor- und Zurück-Antriebsbewegung mit einer Hublänge bereitzustellen; und eine Hautstecheinrichtung, die zumindest teilweise in dem Modulgehäuse angeordnet ist und wenigstens eine Stechnadel an einer Nadelaufnahme sowie eine modulseitige Verbindungsstelle aufweist, welche mit der handstückseitigen Verbindungsstelle verbunden ist. Die Vor- und Zurück-Antriebsbewegung ist über die modulseitige Verbindungsstelle auf die Nadelaufnahme übertragbar, derart, dass im Betrieb die wenigstens eine Stechnadel zwischen einer hinteren Stellung und einer vorderen Stellung verlagerbar ist, in welcher eine Nadelspitze der wenigstens einen Stechnadel gegenüber der Modulgehäuseöffnung im Umfang eines Nadelherausstands übersteht. Das Modulgehäuse mit der Modulgehäuseöffnung ist relativ zum Handstück-Gehäuse zwischen einer ersten und einer zweiten Drehstellung drehbar, und ein erster Nadelherausstand in der ersten Drehstellung des Modulgehäuses ist gleich einem zweiten Nadelherausstand in der zweiten Drehstellung des Modulgehäuses.

Das Handgerät kann an unterschiedliche Anwendungsfälle flexibel und mit geringem Aufwand angepasst werden, insbesondere auch während des Betriebs des Handgeräts, indem der Nutzer das Modulgehäuse des Hautstechmoduls mit der hieran gebildeten Modulgehäuseöffnung relativ zum Handstück verdreht oder mittels Drehen verlagert. Der Nutzer kann also das Handstück unverändert in seiner Hand halten und trotzdem das Modulgehäuse gegenüber dem Handstück verdrehen, um so das Handgerät während der Nutzung zum lokalen Aufstechen der Haut anzupassen.

Mittels Verstellen zwischen der ersten und der zweiten Drehstellung des Modulgehäuses kann beispielsweise eine Drehstellung eines Auflagebereiches am Modulgehäuse, welches bei der Verwendung des Handgeräts zum lokalen Aufstechen auf der Haut zur Auflage kommt, relativ zum Handstück verändert werden. Im Unterschied zum Stand der Technik muss der Nutzer hierzu nicht das Handstück selbst in der Hand oder seinen Fingern verdrehen. Vielmehr kann das Handstück unverändert mit der Hand bzw. den Fingern gehalten werden, lediglich die Drehstellung des Modulgehäuses des Hautstechmoduls wird wie gewünscht verändert oder eingestellt.

Beim Ändern der Drehstellung bleibt der Nadelherausstand der Nadelspitze der wenigstens einen Stechnadel gegenüber der Modulgehäuseöffnung unverändert. Der in der ersten Drehstellung vorhandene Nadelherausstand bleibt also beim Verlagern des Modulgehäuses in die zweite Drehstellung erhalten, so dass bezüglich des Nadelheraus- oder Nadelüberstands nicht veränderte Arbeitsbedingungen an dem Handgerät gegeben sind. Das Verdrehen des Modulgehäuses relativ zum Handstück ist insoweit entkoppelt von einer Änderung des Nadelherausstands.

Das Drehen des Modulgehäuses zwischen der ersten und der zweiten Drehstellung kann um eine Drehachse erfolgen, die in Längsrichtung zumindest des Hautstechmoduls verläuft, die ihrerseits wahlweise gleich einer Längsrichtung des Handstücks ist, insbesondere des Handstück-Gehäuses.

Das Modulgehäuse kann relativ zum Handstück um 360 Grad oder einen kleineren Winkel drehbar sein, beispielweise wenigstens etwa 90 Grad.

Die wenigstens eine Stechnadel kann als eine Einzelnadel oder mit einem Stechnadelbündel gebildet sein. Die wenigstens eine Stechnadel besteht bevorzugt aus einem Vollmaterial (im Unterschied zu einer Kanüle) und ist wahlweise biegesteif ausgebildet, derart, dass sich die Stechnadel beim Aufstechen der Haut im Betrieb unwesentlich oder gar nicht biegt.

Das Hautstechmodul kann mit dem Handstück lösbar verbunden sein. Die modulseitige und die handstückseitige Verbindungsstelle sind dann lösbar miteinander verbunden, sei es direkt oder über ein oder mehrere Verbindungsbauteile.

Bei dieser oder anderen Ausführungsformen kann die Verbindungsstelle an der Nadelaufnahme gebildet sein.

Das Hautstechmodul kann in Bezug auf das Handstück zwischen einer gekoppelten Stellung, in welcher das Hautstechmodul mit dem Handstück funktional oder betreibbar verbunden ist, und einer entkoppelten Stellung verlagerbar sein, in welcher das Hautstechmodul von dem Handstück getrennt ist. In der gekoppelten Stellung kann das Hautstechmodul an dem Handstück fixiert oder gesichert sein, derart, dass es nicht unbeabsichtigt vom Handstück lösbar ist. Die funktionale Verbindung bedeutet, dass in der zugeordneten gekoppelten Stellung die Vor- und Zurück-Antriebsbewegung zwischen den Verbindungsstellen funktionsgerecht zum Betrieb der Hautstecheinrichtung übertragbar ist.

Das Modulgehäuse mit der Modulgehäuseöffnung kann relativ zum Handstück-Gehäuse zwischen der ersten und der zweiten Drehstellung stufenlos in weitere Drehstellungen drehbar oder verlagerbar sein, in denen der Nadelherausstand jeweils gleich dem ersten Nadelherausstand ist. Alternativ zur stufenlosen Verstellbarkeit (mittels Drehen) kann vorgesehen sein, dass das Modulgehäuse relativ zum Handstück (zum Handstück-Gehäuse) in voneinander beabstandete Drehstellungen verlagerbar ist. Es kann vorgesehen sein, dass das Modulgehäuse in den verschiedenen Drehstellungen gegen ein unbeabsichtigtes Verdrehen gesichert ist, beispielsweise mittels einer Rastung und / oder einer Klemmung in der jeweiligen Drehstellung, die vom Nutzer wahlweise mittels geringem Kraftaufwand überwindbar ist, um das Modulgehäuse zu verdrehen. Eine solche Rastung kann eingerichtet sein, ein rein haptisches Feedback beim Drehen gegen einen praktisch unmerklichen Widerstand bereitzustellen.

Der Nadelherausstand kann mittels einer Einstelleinrichtung veränderbar oder einstellbar sein. Mit Hilfe der Einstelleinrichtung kann das Ausmaß oder der Umfang des Nadelherausstands verändert werden, also der Herausstand oder Überstand der Nadelspitze der wenigstens einen Stechnadel gegenüber der Modulgehäuseöffnung in der ausgefahrenen Stellung der Stecheinrichtung, beispielsweise im laufenden Betrieb. Der Nadelherausstand kann hierbei zum Beispiel als der Abstand zwischen einer Stechspitze im Bereich der Nadelspitze und einem demgegenüber zurückversetzten Randabschnitt eines die Modulgehäuseöffnung umgebenden Modulabschnitts des Hautstechmoduls bestimmt sein. Der Nadelherausstand kann mit Hilfe der Einstelleinrichtung stufenlos oder in gestuften Abständen einstellbar sein.

Ist die wenigstens eine Stechnadel in die hintere Stellung verlagert, kann die Nadelspitze der wenigstens einen Stechnadel hinter die Modulgehäuseöffnung zurücktreten oder, im Vergleich zur vorderen Stellung, in geringerem Umfang über die Modulgehäuseöffnung überstehen.

Für mehrere verschieden eingestellte Nadelherausstände können jeweils der erste Nadelherausstand in der ersten Drehstellung des Modulgehäuses gleich dem zweiten Nadelherausstand in der zweiten Drehstellung des Modulgehäuses sein, wenn das Modulgehäuse mit der Modulgehäuseöffnung relativ zum Handstück-Gehäuse zwischen der ersten und der zweiten Drehstellung verlagert wird. Für unterschiedliche Nadelherausstände, die mit Hilfe der Einstelleinrichtung (zuvor) eingestellt werden, bleibt der so eingestellte Nadelherausstand jeweils erhalten, also unverändert, wenn das Modulgehäuse mit der Modulgehäuseöffnung relativ zum Handstück-Gehäuse zwischen der ersten und der zweiten Drehstellung verlagert wird. Der Nutzer kann also mit Hilfe der Einstelleinrichtung einen gewünschten Nadelherausstand einstellen, der dann erhalten bleibt, wenn das Modulgehäuse relativ zum Handstück-Gehäuse verdreht wird (unterschiedliche Drehstellungen). Das Verlagern zwischen den unterschiedlichen Drehstellungen des Modulgehäuses, nachdem Modulgehäuse und Handstück-Gehäuse zuvor miteinander verbunden wurden, ist zu unterscheiden von einer möglichen Relativbewegung zwischen Modulgehäuse und Handstück-Gehäuse beim Andocken des Modulgehäuses an das Handstück-Gehäuse, also beim Verbinden von Hautstechmodul und Handstück.

Es kann ein Griffstück vorgesehen sein, welches mit dem Hautstechmodul drehfest und dem Handstück verdrehbar verbunden ist, wenn das Handstück und das Hautstechmodul verbunden sind. Mit Hilfe des Griffstücks oder -bauteils kann der Nutzer das Hautstechmodul drehen, wohingegen das Handstück, also insbesondere das Handstück-Gehäuse demgegenüber feststehend verbleiben kann, sich also beim Drehen des Griffstücks nicht mit dreht. Das Griffstück kann insbesondere an dem Modulgehäuse drehfest angeordnet sein. In Bezug auf das Handstück-Gehäuse ist das Griffstück (zusammen mit dem Modulgehäuse) demgegenüber verdrehbar.

Das Griffstück kann mittels einer ersten mechanischen Verbindung mit dem Handstück verbunden sein. Die erste mechanische Verbindung kann zum Beispiel mit einer Rast-, Schraub-, Klemm- und / oder einer Klickverbindung gebildet sein. Die erste mechanische Verbindung kann zwischen dem Griffstück und dem Handstück-Gehäuse ausgebildet sein.

Das Griffstück kann mittels einer zweiten mechanischen Verbindung mit dem Hautstechmodul verbunden sein. Die zweite mechanische Verbindung kann beispielsweise als Rast-, Schraub-, Klemm- und / oder einer Klickverbindung ausgeführt sein. Das Griffstück kann hierbei mit dem Modulgehäuse über die zweite mechanische Verbindung verbunden sein.

Das Griffstück kann mit dem Hautstechmodul und / oder dem Handstück lösbar verbunden sein. Insbesondere kann das Griffstück mit dem Modulgehäuse und / oder dem Handstück-Gehäuse lösbar verbunden sein.

Bei dieser oder anderen Ausführungsformen kann das Griffstück entlang einer äußeren (umlaufenden) Oberfläche Griffelemente aufweisen, beispielsweise Griffmulden oder Griffvorsprünge, die eingerichtet sind, vom Nutzer zum Verlagern des Griffstücks mit einem oder mehreren Fingern gegriffen zu werden.

Die erste mechanische Verbindung zwischen dem Griffstück und dem Handstück kann gegen ein Trennen der ersten mechanischen Verbindung gesichert sein, wenn oder nachdem das Griffstück und das Hautstechmodul mittels der zweiten mechanischen Verbindung verbunden sind. Das Ausbilden der zweiten mechanischen Verbindung zwischen dem Griffstück und dem Hautstechmodul, insbesondere zwischen dem Griffstück und dem Modulgehäuse, sichert hierbei die erste mechanische Verbindung zwischen Griffstück und Handstück. Nach dem Ausbilden der zweiten mechanischen Verbindung ist ein Trennen der ersten mechanischen Verbindung zwischen Griffstück und Handstück nicht (mehr) möglich.

Ist die zweite mechanische Verbindung (noch) nicht ausgebildet, kann vorgesehen sein, dass in diesem Zustand die erste mechanische Verbindung (wieder) lösbar ist, um Handstück und Griffstück zu trennen. Das bedeutet, dass zu diesem Zeitpunkt das Griffstück noch vom Handstück abgenommen werden kann, was nach dem Herstellen der zweiten mechanischen Verbindung nicht mehr möglich ist.

In einer Ausführungsform kann vorgesehen sein, dass an dem Griffstück, von einem vorderen Abschnitt ausgehend, nach hinten zum Handstück hin vorspringende federnde Rasthaken angeordnet sind, die beim Aufsetzen des Griffstücks auf einen zugeordneten Gehäuseabschnitt des Handstück-Gehäuses mit einer zugeordneten Vertiefung oder einem zugeordneten Vorsprung zum Eingriff kommen. Wird sodann die zweite mechanische Verbindung ausgebildet, kann vorgesehen sein, dass die Rasthaken durch das angekoppelte Hautstechmodul radial mit einer Spannkraft beaufschlagt werden, so dass ein Lösen der Rasthaken aus der zugeordneten Aufnahme (Vertiefung oder Vorsprung) nicht mehr möglich ist, was zuvor, also vor dem Ausbilden der zweiten mechanischen Verbindung, zum Lösen des Griffstücks vom Handstück möglich war. Soll später das Griffstück wieder vom Handstück gelöst werden, ist bei diesem Beispiel zunächst die zweite mechanische Verbindung zwischen dem Griffstück und dem Hautstechmodul zu trennen, bevor dann das Griffstück von dem Handstück gelöst oder getrennt werden kann.

Das Griffstück kann mit einer auf einen Gehäuseabschnitt des Handstück-Gehäuse aufsteckbaren Griffhülse gebildet sein. An der Griffhülse können beispielsweise die Rasthaken aufweisen, zum Beispiel innenseitig umlaufend.

Die Modulgehäuseöffnung kann eine Öffnungsfläche aufweisen, die in der ersten Drehstellung eine erste Relativlage zum Handstück-Gehäuse und in der zweiten Drehstellung eine zweite Relativlage zum Handstück-Gehäuse aufweist, welche von der ersten Relativlage verschieden ist. In der Öffnungsfläche der Modulgehäuseöffnung bewegt sich die wenigstens eine Stechnadel im Betrieb aufgrund der Vor- und Zurück-Antriebsbewegung.

Die Öffnungsfläche kann schräg zur Arbeitsrichtung der Antriebsbewegung ausgebildet sein.

An einem vorderen Gehäuseende des Modulgehäuses kann benachbart zur Modulgehäuseöffnung ein Auflagebereich ausgebildet sein, beispielsweise eine gerade oder eine gekrümmte Kante, welcher im Betrieb auf der lokal aufzustechenden Haut zur Auflage kommt. Mittels Drehen des Modulgehäuses ist die Relativlage des Auflagebereichs in Bezug auf das Handstück sowie die Arbeitsrichtung veränderbar.

Die Hublänge kann mittels einer Hubeinstelleinrichtung veränderbar sein. Mit Hilfe der Hubeinstelleinrichtung wird der Weg (Hub) zwischen der vorderen und der hinteren Stellung beim Bewegen der Nadelaufnahme mit der wenigstens einen Stechnadel verändert. Die Hubeinstelleinrichtung kann in dem Handstück-Gehäuse angeordnet sein. Die Hubeinstellung kann getrennt oder entkoppelt von dem Einstellen des Nadelherausstands ausführbar sein. Auch kann vorgesehen sein, dass eine eingestellte Hubeinstellung unverändert bleibt, wenn das Modulgehäuse zwischen der ersten und der zweiten Drehstellung verlagert wird. Bei dieser Ausführungsform sind sowohl der Nadelherausstand wie auch die Hublänge unabhängig von dem Verlagern des Modulgehäuses relativ zum Handstück, verändern sich also nicht, wenn das Modulgehäuse zwischen der ersten und der zweiten Drehstellung (oder in weitere Drehstellungen) verlagert wird.

Die Hubeinstelleinrichtung kann eingerichtet sein, zum Einstellen der Hublänge eine Relativlage zwischen der Nadelaufnahme mit der wenigstens einen Stechnadel und der Modulgehäuseöffnung entlang der Arbeitsrichtung einzustellen.

Nach einem anderen Aspekt kann eine Antriebsvorrichtung für ein Handgerät zum lokalen Aufstechen einer Haut vorgesehen sein, welche Folgendes aufweist: ein Gehäuse, welches mit einem Hautstechmodul verbindbar ist; eine Antriebseinrichtung, die in dem Gehäuse angeordnet und eingerichtet ist, eine Antriebskraft mittels einer drehenden Antriebsbewegung einer Antriebswelle bereitzustellen, deren Axialrichtung quer zu einer Arbeitsrichtung verläuft; ein Verbindungsbauteil, welches eingerichtet ist, getrieben von der Antriebskraft, entlang der Arbeitsrichtung eine Vor- und Zurückbewegung (Vor- und Zurück-Antriebsbewegung) mit einer Hublänge auszuführen; eine (wahlweise handstückseitige) Verbindungsstelle, die an dem Verbindungsbauteil gebildet und zum Verbinden mit einer Hautstecheinrichtung des Hautstechmoduls eingerichtet ist; ein Wandlungsmechanismus, welcher, die Antriebskraft übertragend, zwischen der Antriebswelle und dem Verbindungsbauteil in dem Gehäuse angeordnet ist und eine Schwenkhebelanordnung aufweist, wobei bei dem Wandlungsmechanismus die Schwenkhebelanordnung mit einem Schwenkhebel gebildet ist, mit dem die drehende Antriebsbewegung der Antriebswelle abgreifbar ist und der hierbei um einen Drehpunkt schwenkt, der in Bezug auf die Antriebswelle radial versetzt ist, und ein Pleuelbauteil auf einer Seite mit einem Abgriff an dem Schwenkhebel und gegenüberliegend mit dem Verbindungsbauteil verbunden ist; und eine Hubeinstelleinrichtung, mit der zum Einstellen der Hublänge der Vor- und Zurückbewegung entlang der Arbeitsrichtung der Abgriff an dem Schwenkhebel verlagerbar ist. Nach einem weiteren Aspekt kann ein Handgerät zum lokalen Aufstechen einer Haut mit der Antriebsvorrichtung sowie dem Hautstechmodul vorgesehen sein, welches mit der Antriebsvorrichtung verbunden ist.

Mit der Antriebsvorrichtung kann das Handstück für das Handgerät zum lokalen Aufstechen einer menschlichen oder einer tierischen Haut gebildet sein. Mit dem Gehäuse der Antriebsvorrichtung kann dann eine Handstück-Gehäuse gebildet sein.

Mit Hilfe der Antriebsvorrichtung wird bei diesem Beispiel eine Antriebskraft zur Abgabe über das Verbindungsbauteil bereitgestellt, derart, dass die Antriebskraft über das Verbindungsbauteil auf eine Hautstecheinrichtung eines Hautstechmoduls, welches mit der Antriebsvorrichtung verbindbar ist, eingeleitet werden kann, um ein oder mehrere Stechnadeln der Hautstecheinrichtung zum lokalen Aufstechen einer Haut vor- und zurückzubewegen. Die bereitgestellte Vor- und Zurückbewegung ist durch eine Hublänge charakterisiert, also einen Abstand zwischen vorderem und hinteren Endpunkt der Vor- und Zurückbewegung (Vor- und Zurück-Antriebsbewegung) entlang der Arbeitsrichtung. Wird die bereitgestellte Antriebsbewegung entlang der Arbeitsrichtung unverändert auf die Hautstecheinrichtung übertragen, führt dies im Betrieb zu einer Vor- und Zurückbewegung der Hautstecheinrichtung mit einer Arbeitshublänge, welche der mittels der Antriebsvorrichtung bereitgestellten Hublänge entspricht. Der Arbeitshub wird von der Hautstecheinrichtung, insbesondere den hiervon umfassten Stechnadeln, im Betrieb ausgeführt. Mittels einer optionalen Hubumsetzung kann die Arbeitshublänge wahlweise kürzer oder länger als die Hublänge ausgebildet werden.

Die von der Antriebsvorrichtung bereitgestellte Hublänge ist mit Hilfe der Hubeinstelleinrichtung veränderbar. Hierzu ist bei dem Beispiel an dem Schwenkhebel der Abgriff verlagerbar. So ist auf einfache und zuverlässige Art und Weise eine Einstellung der Hublänge, die mit Hilfe der Antriebsvorrichtung bereitgestellt wird, für unterschiedliche Anwendungen beim lokalen Aufstechen der Haut ermöglicht.

Die (handstückseitige) Verbindungsstelle kann für ein gelenkige Verbindung des Verbindungsbauteils mit der modulseitigen Verbindungsstelle eingerichtet sein. Beispielsweise kann an dem Verbindungsbauteil im Bereich der Verbindungsstelle ein Kugelkopfgelenkbauteil vorgesehen sein. Die Verbindungsstelle kann in einer Führung angeordnet sein, die sich in der Arbeitsrichtung erstreckt.

Die Antriebseinrichtung kann eine maschinelle Antriebseinrichtung sein, die mit einem elektrischen Motor ausgeführt ist, der zum Beispiel als ein Außenläufer-Motor ausgeführt sein kann.

Die Antriebsvorrichtung kann eine Messeinrichtung aufweisen, die der Hubeinstelleinrichtung zugeordnet und eingerichtet ist, unterschiedliche Verlagerungsstellungen des Abgriffs an dem Schwenkhebel zu erfassen. Mit Hilfe der Messeinrichtung werden Messwerte erfasst, die die unterschiedlichen Verlagerungsstellungen des Abgriffs an dem Schwenkhebel anzeigen. Beispielsweise können elektronische Messwerte erfasst werden, zum Beispiel unter Verwendung eines Potentiometers oder eines anderen Messwertgebers, dessen unterschiedliche Stellungen den verschiedenen Verlagerungsstellungen des Abgriffs entsprechen.

An dem Gehäuse der Antriebsvorrichtung kann eine Anzeigeeinrichtung vorgesehen sein, welche die verschiedenen Verlagerungsstellungen des Abgriffs anzeigt. Die Anzeige kann hierbei die mittels der Messeinrichtung erfassten Messwerte aufgreifen und anzeigen. Alternativ oder in Ergänzung zur Messeinrichtung kann eine mechanische Anzeigeeinrichtung vorgesehen sein, beispielsweise ein Drehbauteil mit Anzeigeelementen entlang der umlaufenden Oberfläche, mit dem die verschiedenen Verlagerungsstellungen des Abgriffs an dem Schwenkhebel an der Antriebsvorrichtung dem Benutzer angezeigt werden.

Die Hubeinstelleinrichtung kann bei den verschiedenen Beispielen des Handgeräts mit einer manuellen Hubeinstelleinrichtung gebildet sein. Die manuelle Hubeinstelleinrichtung ermöglicht es dem Benutzer, den Abgriff an dem Schwenkhebel zum Einstellen der Hublänge mittels manueller Bedienung zu bewirken. Beispielsweise kann am Gehäuse der Antriebsvorrichtdung ein Einstellrad oder ein Schiebeelement vorgesehen sein.

Die Hubeinstelleinrichtung kann mit einer elektronischen Hubeinstelleinrichtung gebildet sein. In Verbindung mit der elektronischen Hubeinstellung kann die Verlagerung des Abgriffs an dem Schwenkhebel mit Hilfe von elektronischen Steuersignalen an die Hubeinstelleinrichtung bewirkt werden. Diese kann hierzu zum Beispiel einen Schrittmotor umfassen, mit dem die Stellung des Abgriffs an dem Schwenkhebel in verschiedene Positionen relativ zum Schwenkhebel verlagert werden kann.

Die elektronischen Hubeinstelleinrichtung kann bei den verschiedenen Beispielen des Handgeräts mit einer Steuereinrichtung verbunden sein, derart, dass von der Steuereinrichtung bereitstellbare Hubeinstellsignale an die elektronische Hubeinstelleinrichtung übertragbar sind. Die Steuereinrichtung kann in oder an dem Gehäuse der Antriebsvorrichtung angeordnet sein. Alternativ kann die Steuereinrichtung getrennt von der Antriebsvorrichtung gebildet sein und mit der elektronischen Hubeinstelleinrichtung über eine kabelgebundene oder kabellose Verbindung Steuersignale austauschen. Die Steuereinrichtung kann eingerichtet sein, auf eine Benutzerauswahl die elektronische Hubeinstelleinrichtung anzusteuern, so dass eine vom Benutzer vorgewählte Hublänge eingestellt wird.

Die Hubeinstelleinrichtung kann bei den verschiedenen Beispielen derart eingerichtet sein, dass der Abgriff an dem Schwenkhebel verlagerbar ist, während die drehende Antriebsbewegung über die Antriebswelle bereitgestellt wird. Bei dieser Ausführungsform ist es ermöglicht, die Hublänge der Vor- und Zurückbewegung während des Betriebs der Antriebsvorrichtung zu verändern, also während die drehende Antriebsbewegung von der Antriebseinrichtung bereitgestellt wird. Dieses unterstützt eine schnelle und flexible Änderung der Hublänge während des Betriebs der Antriebsvorrichtung.

Die Hubeinstelleinrichtung kann bei den verschiedenen Beispielen eingerichtet sein, beim Verlagern des Abgriffs an dem Schwenkhebel einen Abstand des Abgriffs zum Drehpunkt des Schwenkhebels zu ändern.

Die Antriebsvorrichtung kann eine Abgriffeinrichtung zum Abgreifen der drehenden Antriebsbewegung der Antriebswelle aufweisen, wobei die Abgriffeinrichtung ein Drehbauteil, welches mit der Antriebswelle drehfest verbunden ist, und ein mit dem Drehbauteil in Verbindung stehendes erstes Gleitbauteil aufweist, welches in einer ersten Gleitführung an dem Schwenkhebel verlagerbar angeordnet ist. Das erste Gleitbauteil kann an einem Kurbelzapfen angeordnet sein, welcher an Drehbauteil (exzentrisch in Bezug auf die Antriebswelle) angeordnet ist, zum Beispiel aufrecht stehend auf einer Oberfläche des Drehbauteils, die ihrerseits quer zur Axialrichtung der Antriebswelle steht. Der Kurbelzapfen ist in radialer Richtung versetzt zur Antriebswelle angeordnet. Das Drehbauteil kann Teil der Antriebseinrichtung sein, insbesondere des elektrischen Motors.

Mit Hilfe der Abgriffeinrichtung wird die über die Antriebswelle bereitgestellte drehende Antriebsbewegung abgegriffen. Das Drehbauteil dreht sich beim Drehen der Antriebswelle mit dieser, wodurch der Kurbelzapfen umläuft. Mit Hilfe des mit dem Drehbauteil in Verbindung stehenden ersten Gleitbauteils (am Kurbelzapfen) in der ersten Gleitführung an dem Schwenkhebel wird der Schwenkhebel aufgrund der drehenden Antriebsbewegung der Antriebswelle (und des Drehbauteils sowie des exzentrischen Kurbelzapfens) in eine Schwenkbewegung um den Drehpunkt versetzt. Das erste Gleitbauteil kann in der ersten Gleitführung entlang einer geraden oder einer gekrümmten Bewegungs- oder Gleitbahn geführt werden.

Der Abgriff kann an dem Schwenkhebel mit einem zweiten Gleitbauteil gebildet sein, welches in einer zweiten Gleitführung an dem Schwenkhebel verlagerbar angeordnet ist. Bei dieser Ausführungsform ist im Zusammenhang mit dem Abgriff an dem Schwenkhebel das zweite Gleitbauteil in der zweiten Gleitführung angeordnet, die an dem Schwenkhebel ausgebildet ist, insbesondere als von der ersten Gleitführung getrennt ausgeführte weitere Gleitführung. In der zweiten Gleitführung kann das zweite Gleitbauteil entlang einer geraden oder einer gekrümmten Bewegungsbahn hin und her bewegt werden.

Eine Längsrichtung der ersten Gleitführung und eine Längsrichtung der zweiten Gleitführung können einen spitzen Winkel einschließen. Ist zumindest eine der Gleitführungen als eine gekrümmte Gleitführung ausgeführt, so entspricht die Längsrichtung der gekrümmten Gleitführung der geraden Verbindung zwischen den gegenüberliegenden Enden der Gleitführung.

Das Pleuelbauteil und das zweite Gleitbauteil können im Bereich des Abgriffs relativ zueinander um eine Schwenkachse schwenkbar sein. Das Pleuelbauteil und das zweite Gleitbauteil können zum Beispiel mittels einer Stiftlagerung miteinander verbunden sein. Die Schwenkachse kann im Wesentlichen parallel zur Axialrichtung der Antriebswelle verlaufen.

Die Hubeinstelleinrichtung kann bei den verschiedenen Beispielen des Handgeräts ein Einstellbauteil aufweisen, bei dem ein der Hubeinstelleinrichtung zugeordnetes und mit dem Abgriff verbundenes drittes Gleitbauteil in einer dritten Gleitführung an dem Einstellbauteil verlagerbar angeordnet ist. Das Einstellbauteil ist als Bestandteil der Hubeinstelleinrichtung verlagerbar, um zum Einstellen der Hublänge den Abgriff am Schwenkhebel zu verlagern. Führt der Schwenkhebel im Betrieb eine Schwenkbewegung aus, bewegt sich das der Hubeinstelleinrichtung zugeordnete dritte Gleitbauteil in der dritten Gleitführung an dem Einstellbauteil hin und her. Die dritte Gleitführung kann für das dritte Gleitbauteil eine gerade oder eine gekrümmte Führungsbahn bereitstellen. Die Längsrichtung der dritten Gleitführung kann im Wesentlichen quer zur Antriebswelle und / oder zur Arbeitsrichtung verlaufen. Das Einstellbauteil selbst verbleibt nach dem Verlagern in eine gewünschte Stellung (in Verbindung mit einer vorgesehenen Stellung des Abgriffs am Schwenkhebel) ortsfest, wenn die Antriebseinrichtung betrieben wird, es sei denn, die Hublänge soll geändert werden, was mit Hilfe einer Verlagerung des Einstellbauteils ermöglicht ist, wahlweise auch im laufenden Betrieb.

Das Pleuelbauteil und das der Hubeinstelleinrichtung zugeordnete dritte Gleitbauteil können im Bereich des Abgriffs relativ zueinander um die Schwenkachse schwenkbar sein.

Der Wandlungsmechanismus zum Übertragen der Antriebsbewegung kann frei von einer gemeinsamen Drehachse zwischen zweitem und drittem Gleitbauteil gebildet sein. Es kann alternativ eine gelenkige Verbindung anderswo vorgesehen sein, oder sogar ganz entfallen. Stattdessen kann eine andere Form der Kopplung vorgesehen sein, beispielsweise ein variabel seitlich versetztes Schieberbauteil, ein elastisches Pleueldrahtbauteil oder dergleichen.

Die Arbeitsrichtung kann in Längsrichtung des Gehäuses ausgebildet sein.

Die vorangehend im Zusammenhang mit der Antriebsvorrichtdung erläuterten Ausgestaltungen können in Verbindung mit dem Handgerät zum lokalen Aufstechen der Haut entsprechend vorgesehen sein.

Bei dem Handgerät kann das Hautstechmodul, bei dem in einem Modulgehäuse die Hautstecheinrichtung mit der einen oder der mehreren Stechnadeln angeordnet ist, lösbar oder nicht lösbar verbunden sein. Im Fall einer lösbaren Montage des Hautstechmoduls an der Antriebsvorrichtung, insbesondere an deren Gehäuse, kann das Hautstechmodul als ein Einwegmodul ausgeführt sein.

Mit dem Gehäuse der Antriebsvorrichtung kann ein Handstücks des Handgeräts bereitgestellt sein, welches vom Benutzer mit den Fingern gegriffen wird, um das Handgerät zum lokalen Aufstechen einer Haut zu führen.

Die maschinelle Antriebseinrichtung, beispielsweise der elektrische Motor, wird über eine kabelgebundene oder eine kabellose Verbindung mit elektrischer Energie versorgt, um im Betrieb die drehende Antriebsbewegung bereitzustellen.

### Beschreibung von Ausführungsbeispielen

Im Folgenden werden weitere Ausführungsbeispiele unter Bezugnahme auf Figuren einer Zeichnung erläutert. Hierbei zeigen:
- Fig. 1: eine schematische Darstellung eines Handgeräts zum lokalen Aufstechen einer Haut;
- Fig. 2: eine schematische perspektivische Darstellung des Handgeräts aus Fig. 1;
- Fig. 3: eine schematische perspektivische Darstellung des Handgeräts aus Fig. 1, wobei ein Hautstechmodul von einer Antriebsvorrichtung gelöst ist;
- Fig. 4: eine schematische Darstellung des Handgeräts aus Fig. 1, wobei ein Teil eines Gehäuses einer Antriebsvorrichtung weggelassen ist;
- Fig. 5: eine schematische perspektivische Darstellung des Handgeräts aus Fig. 1 teilweise im Schnitt und ohne Gehäuse;
- Fig. 6: eine schematische Darstellung eines Wandlungsmechanismus des Handgeräts nach Fig. 1;
- Fig. 7: eine weitere schematische Darstellung des Wandlungsmechanismus aus Fig. 6 mit einer ersten Einstellung einer Hubeinstelleinrichtung;
- Fig. 8: eine weitere schematische Darstellung des Wandlungsmechanismus aus Fig. 6 mit einer zweiten Einstellung einer Hubeinstelleinrichtung und
- Fig. 9: eine schematische perspektivische Darstellung von Elementen des Wandlungsmechanismus und einer Antriebseinrichtung;
- Fig. 10: eine schematische perspektivische Darstellung des Handgeräts zum lokalen Aufstechen einer Haut, wobei Hautstechmodul und Griffstück vom Handstück getrennt sind;
- Fig. 11: eine schematische perspektivische Darstellung des Handgeräts aus Fig. 10 teilweise im Schnitt;
- Fig. 12: eine schematische perspektivische Darstellung des Handgeräts aus Fig. 11, wobei das Griffstück mit dem Handstück verbunden ist;
- Fig. 13: eine schematische perspektivische Darstellung des Handgeräts aus Fig. 11, wobei Hautstechmodul, Griffstück und Handstück miteinander verbunden sind,
- Fig. 14: eine schematische perspektivische Detaildarstellung mit einer ersten Einstellung für den Nadelherausstand und
- Fig. 15: eine schematische perspektivische Detaildarstellung mit einer zweiten Einstellung für den Nadelherausstand.

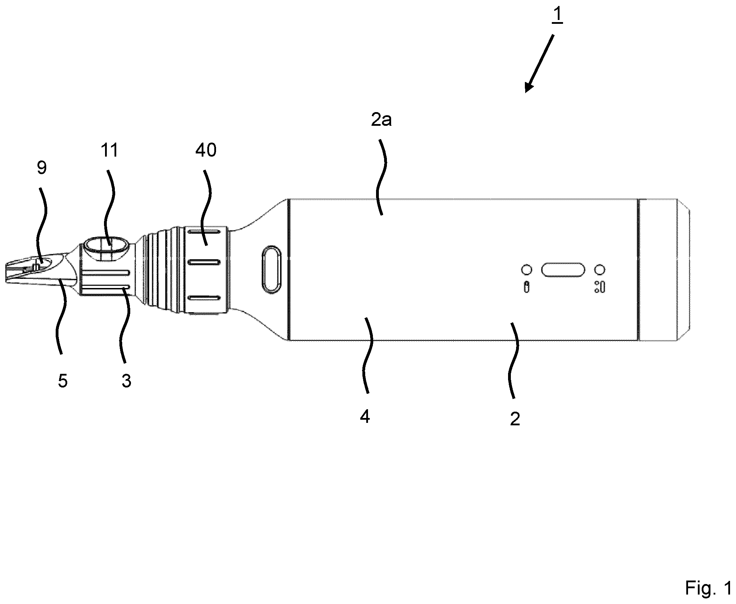

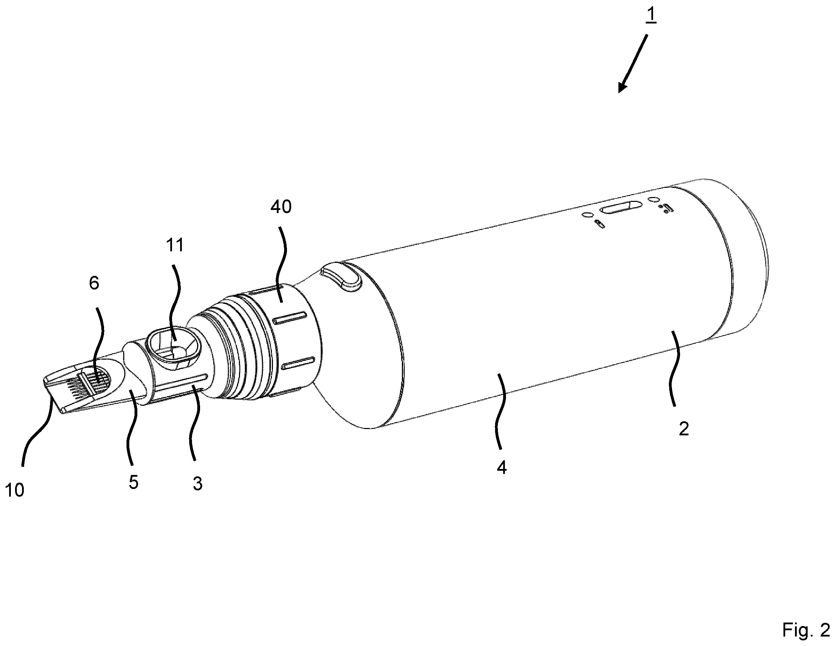

Fig. 1 bis 3 zeigen Darstellungen eines Handgeräts 1 zum lokalen Aufstechen einer menschlichen oder einer tierischen Haut. Das Handgerät 1 weist eine Antriebsvorrichtung 2 sowie ein hiermit verbundenes Hautstechmodul 3 auf, welches mit einem Gehäuse 4 der Antriebsvorrichtung 2 lösbar oder nicht lösbar verbunden ist. Ist das Hautstechmodul 3 mit der Antriebsvorrichtung 2 lösbar verbunden, kann es als Einwegmodul ausgeführt sein, so dass das Hautstechmodul 3 nach einer Verwendung entsorgt werden kann. Das Gehäuse 4 der Antriebsvorrichtung 2 wie auch ein Modulgehäuse 5 des Hautstechmoduls 3 können ein- oder mehrstückig ausgebildet sein.

Mit der Antriebsvorrichtung 2 kann ein Handstück 2a des Handgeräts 1 zum lokalen Aufstechen einer menschlichen oder einer tierischen Haut gebildet sein, welches vom Nutzer im Betrieb in die Hand oder die Finger genommen wird. Das Gehäuse 4 bildet dann ein Handstück-Gehäuse, welches zum Ausbilden des Handgeräts 1 mit dem Hautstechmodul 3 verbunden wird, insbesondere dessen Modulgehäuse 5.

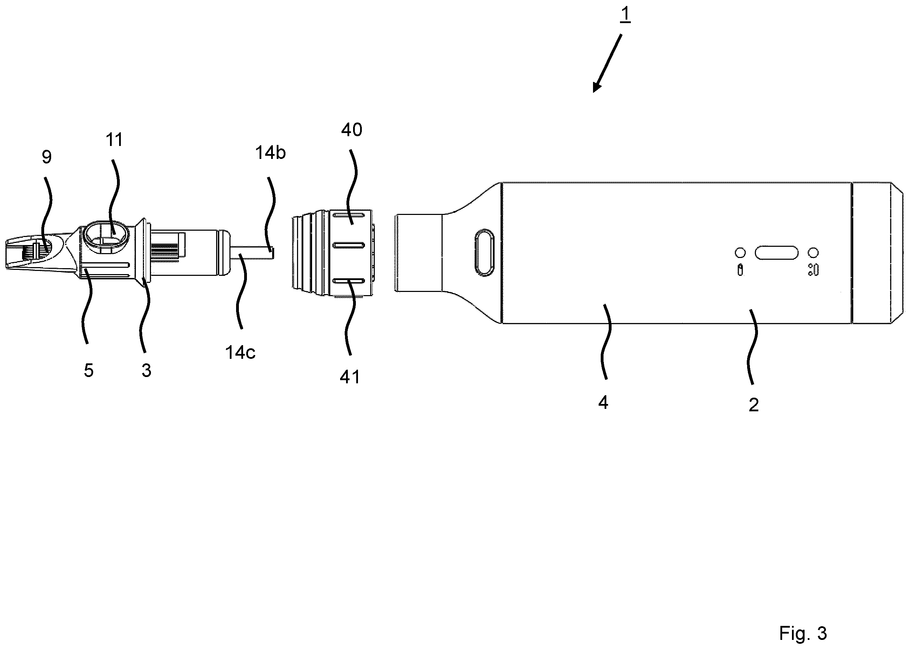

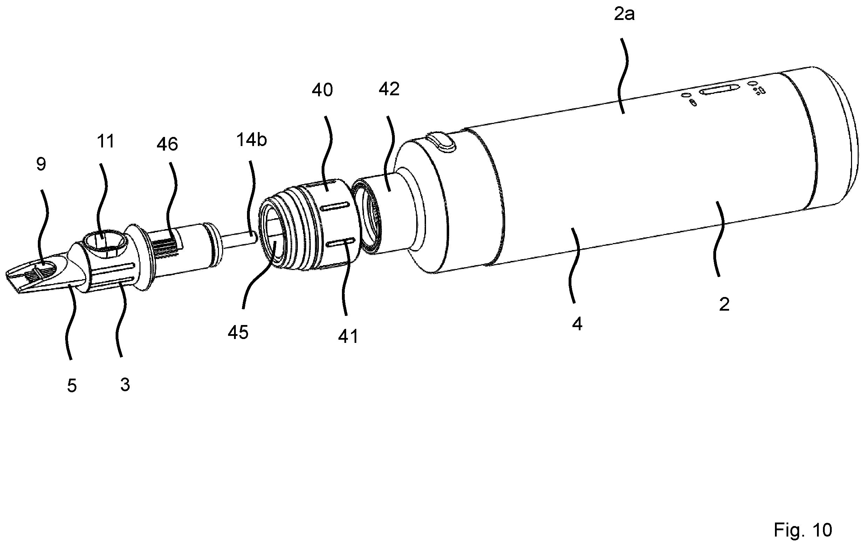

Das Hautstechmodul 3 ist gemäß Fig. 3 von der Antriebsvorrichtung 2 lösbar und somit austauschbar. Bei dem gezeigten Beispiel sind die Antriebsvorrichtung 2, insbesondere das Gehäuse 4, sowie das Hautstechmodul 3, insbesondere das Modulgehäuse 5, mit einem Griffstück 40 verbunden (vgl. weitere Erläuterungen unten zu Fig. 10 bis 15).

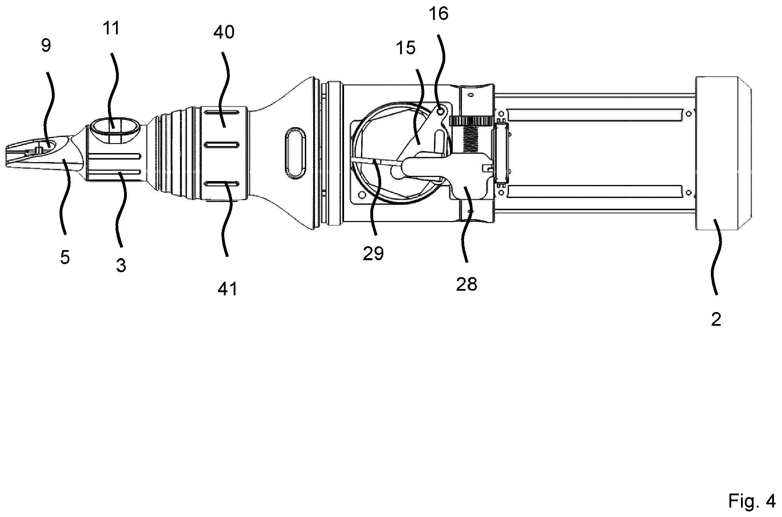

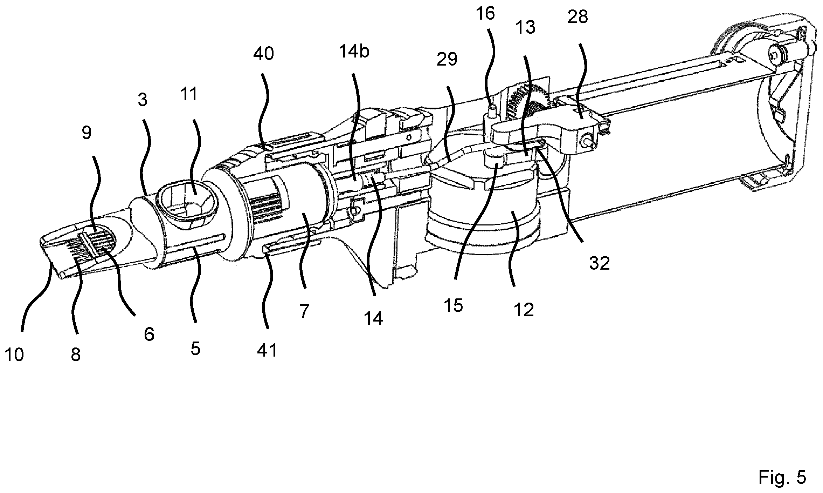

Fig. 4 und 5 zeigen schematische Darstellungen des Handgeräts 1, wobei das Handgerät 1 teilweise ohne das Gehäuse 4 der Antriebsvorrichtung 2 sowie teilweise im Schnitt dargestellt ist.

Zum lokalen Aufstechen einer Haut weist das Hautstechmodul 3 mehrere Stechnadeln 6 an einer Hautstecheinrichtung 7 auf, die im Modulgehäuse 5 des Hautstechmoduls 3 verlagerbar aufgenommen ist. Im Betrieb des Handgeräts 1 werden Nadelspitzen 8 der Stechnadel 6 durch eine vorderseitige Öffnung 9 des Modulgehäuses 5 aus- und eingefahren, derart, dass die Nadelspitzen 8 zumindest in einer ausgefahrenen Stellung (nicht dargestellt) gegenüber einer vorderen Gehäusekante 10 überstehen, so dass die zu behandelnde Haut lokal mittels der Nadelspitzen 8 aufgestochen werden kann. Ein Farbstoff und / oder ein Wirkstoff, die in Verbindung mit dem lokalen Aufstechen auf eine Haut appliziert werden sollen, können über eine Einfüllöffnung 11 am Modulgehäuse 5 zu den Stechnadeln 6 hin eingebracht werden. Alternativ kann am Gehäuse 4 oder am Modulgehäuse 5 zum Beispiel ein Vorratstank (nicht dargestellt) vorgesehen sein, welcher mit den Stechnadeln 6 in Fluidverbindung steht, so dass eine Flüssigkeit aus dem Vorratsbehälter zu den Stechnadeln 6 hin verbracht werden kann.

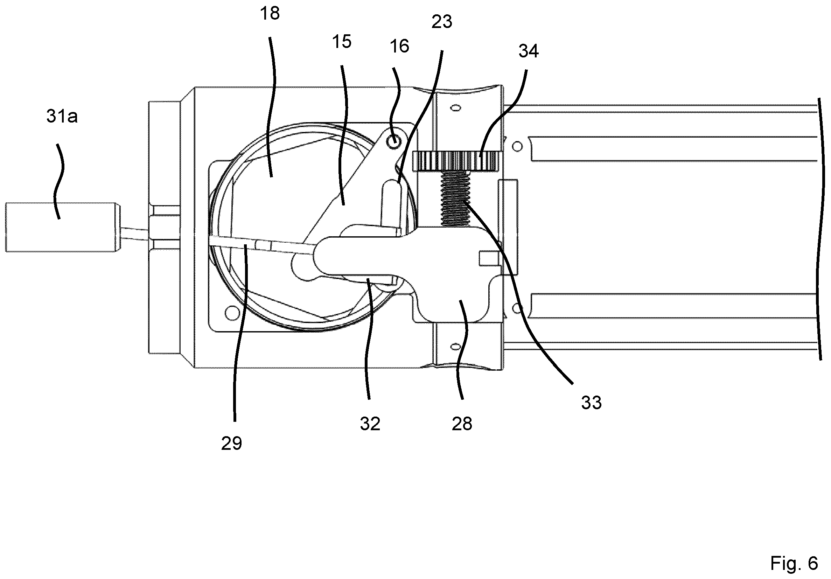

Um die Stechnadeln 6 im Betrieb des Handgeräts 1 in einer Linearbewegung vor- und zurückzubewegen, wird mit Hilfe einer Antriebseinrichtung 12, die beispielsweise mit einem elektrischen Motor gebildet ist, eine drehende Antriebsbewegung bereitgestellt. Mit Hilfe eines Wandlungsmechanismus 13, der nachfolgend insbesondere auch unter Bezugnahme auf die Fig. 6 bis 9 näher erläutert wird, wird eine Antriebskraft übertragen, die drehende Antriebsbewegung in eine Linearbewegung gewandelt und über ein Verbindungsbauteil 14 auf die Hautstecheinrichtung 7 mit den Stechnadeln 6 gekoppelt, so dass die Stechnadeln 6 im Betrieb aufgrund der eingekoppelten Antriebsbewegung eine erzwungene Vor- und Zurückbewegung entlang einer Arbeitsrichtung ausführen, die bei dem gezeigten Ausführungsbeispiel in Längsrichtung des Gehäuses 4 wie auch in Längsrichtung des Modulgehäuses 5 verläuft. An dem Verbindungsbauteil 14 ist eine (beispielweise handstückseitige) Verbindungsstelle 14a vorgesehen, die eingerichtet ist für eine (zum Beispiel lösbare) Verbindung zur Hautstecheinrichtung 7, sei es direkt oder indirekt (vermittelt über ein oder mehrere Zwischenbauteile).

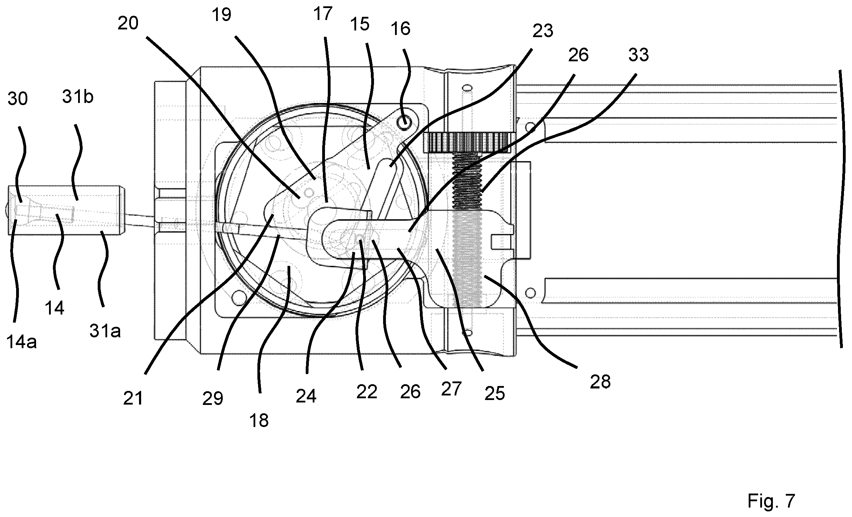

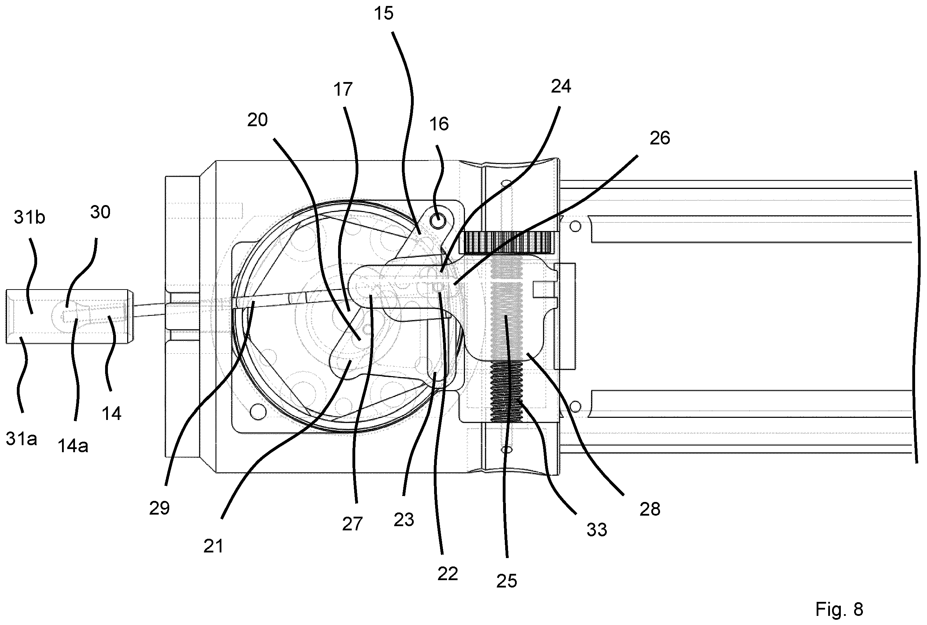

Bei dem Wandlungsmechanismus 13 ist ein Schwenkhebel 15 um einen Drehpunkt 16 schwenkbar gelagert, der in Bezug auf eine Antriebswelle 17 der Antriebseinrichtung 12 radial versetzt angeordnet ist (vgl. Fig. 7 und 8). Die Antriebswelle 17 erstreckt sich bei dem gezeigten Beispiel in einer Axialrichtung quer zur Arbeitsrichtung (also zur Längsrichtung des Gehäuses 4).

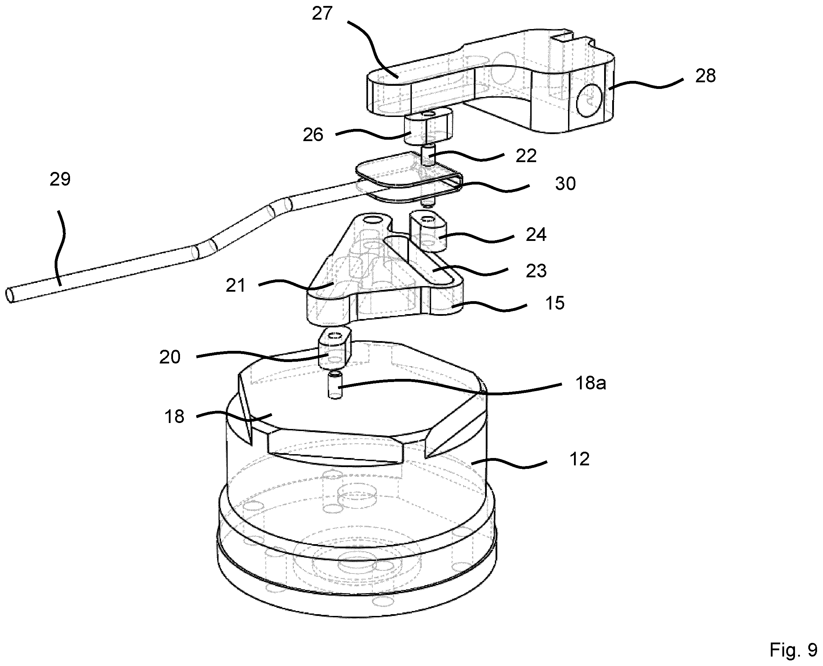

An der Antriebswelle 17 ist ein Drehbauteil 18 drehfest gelagert, so dass sich dieses beim Rotieren der Antriebswelle 17 mit dreht. Drehbauteil 18 ist Teil einer Abgriffeinrichtung 19, bei der das Drehbauteil 18 (zum Beispiel über einen Kurbelzapfen 18a; vgl. Fig. 9) mit einem ersten Gleitbauteil 20 in Verbindung steht, welches seinerseits beim Drehen der Antriebswelle 17 in einer ersten Gleitführung 21 an dem Schwenkhebel 15 bewegt wird. Das erste Gleitbauteil 20 ist an dem Kurbelzapfen 18a drehbar gelagert und bewegt sich in der ersten Gleitführung 21 hin und her, wenn das Drehbauteil 18 mit der Antriebswelle 17 gedreht wird. Auf diese Weise wird die Antriebsbewegung auf den Schwenkhebel 15 übertragen, der dann um den Drehpunkt 16 schwenkt.

An dem Schwenkhebel 15 ist ein Abgriff 22 angeordnet, welcher an dem Schwenkhebel 15 entlang einer zweiten Gleitführung 23 verlagerbar ist. In der zweiten Gleitführung 23 ist ein zweites Gleitbauteil 24 aufgenommen. Die erste und die zweite Gleitführung 21, 23 sind als getrennte Gleitführungen an dem Schwenkhebel 15 gebildet und schließen im gezeigten Beispiel einen spitzen Winkel ein.

Schwenkt der Schwenkhebel 15 aufgrund der Antriebsbewegung um den Drehpunkt 16, bewegt sich ein einer Hubeinstelleinrichtung 25 zugeordnetes drittes Gleitbauteil 26 in einer dritten Gleitführung 27 an einem Einstellbauteil 28 der Hubeinstelleinrichtung 25. Das zweite Gleitbauteil 24 und das der Hubeinstelleinrichtung 25 zugeordnete dritte Gleitbauteil 26 sind an einer gemeinsamen Stiftlagerung 28 relativ zueinander verschwenkbar gelagert. An der Stiftlagerung 28 ist weiterhin ein Pleuelbauteil 29 drehbar gelagert, welches so mit dem Abgriff 22 verbunden ist. Auf einer gegenüberliegenden Seite des Pleuelbauteils 29 ist dieses mit dem Verbindungsbauteil 14 verbunden, welches bei dem dargestellten Ausführungsbeispiel mit einem Kugelkopf 30 gebildet ist. Das Verbindungsbauteil 14 ist für eine gelenkige Verbindung mit der Hautstecheinrichtung 7 im Hautstechmodul 3 eingerichtet. Über das Pleuelbauteil 29 wird schließlich die Antriebsbewegung als lineare Vor- und Zurückbewegung auf die Hautstecheinrichtung 6 übertragen. Das Verbindungsbauteil 14 mit dem Kugelkopf 30 im Bereich der Verbindungsstelle 140 ist beim gezeigten Beispiel in einem Führungsbauteil 30a mit einer Führung 31b aufgenommen und bewegt sich bei der Vor- und Zurückbewegung entlang der Führung 31b (vgl. Fig. 7 und 8).

Die (handstückseitige) Verbindungsstelle 14b ist in dem gezeigten Beispiel an einer rückwärtigen Verlängerung 14c einer die Stechnadeln 6 tragend aufnehmenden Nadelaufnahme angeordnet und eingerichtet, zum Übertragen der Vor- und Zurückbewegung mit einer modulseitigen Verbindungsstelle 14b zusammenzuwirken.

Insbesondere gemäß Fig. 4 ist zwischen dem Schwenkhebel 15 und dem Einstellbauteil 28 im Bereich der Stiftlagerung ein Blechbauteil 32 angeordnet, welches mit dem Pleuelbauteil 29 eine Pleuelbaugruppe bilden kann.

Wie sich insbesondere aus den Fig. 6 bis 9 ergibt, ist das Einstellbauteil 28 der Hubeinstelleinrichtung 25 auf einer Gewindestange 33 angeordnet, so dass das Einstellbauteil 28 mittels Drehen der Gewindestange 33 mit Hilfe eines Einstellrads 34 quer zur Arbeitsrichtung sowie zur Längsrichtung des Gehäuses 4 verlagerbar ist. Auf diese Weise wird der Abgriff 22 am Schwenkhebel 15 verlagert, wobei insbesondere ein Abstand zum Drehpunkt 16 vergrößert oder verkleinert wird. Dieses führt dazu, dass die Hublänge der am Verbindungsbauteil 14 bereitgestellten Vor- und Zurückbewegung in Arbeitsrichtung vergrößert oder verkleinert wird. In den Fig. 6 und 7 sind unterschiedliche Stellungen für das Einstellbauteil 28 entlang der Gewindestange 33 gezeigt, die ihrerseits zu unterschiedlichen Stellungen oder Positionen des Abgriffs 22 an dem Schwenkhebel 15 führen, und damit zu unterschiedlicher Hublänge.

Mittels Wahl eines Winkels zwischen der ersten und der zweiten Gleitführung 21, 23 am Schwenkhebel 15 ergibt sich, dass in einer hinteren Betriebslage (siehe Fig. 8) die zweite Gleitführung 21 auf voller Länge genau quer zur Längsachse zu liegen kommt, was dazu führt, dass für alle Hubeinstellungen der hintere Umkehrpunkt weitestgehend konstant bleibt. Eine vollständige Konstanz kann ausgebildet werden, indem die zweite Gleitführung 23 kreisbogenförmig mit einem Mittelpunkt identisch dem des Kugelkopfes 30 am Verbindungsbauteil 14 gewählt wird.

Fig. 10 bis 15 zeigen weitere schematische perspektivische Darstellungen des Handgeräts 1.

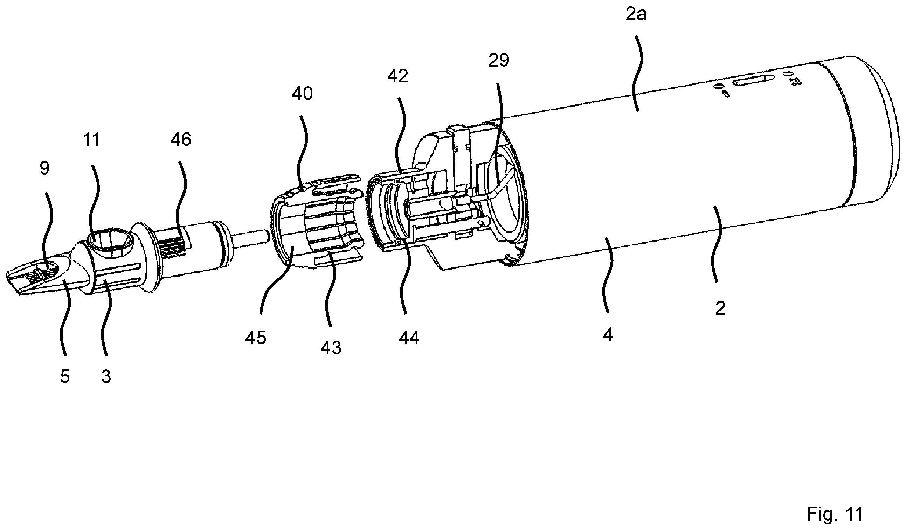

Gemäß Fig. 10 sind bei dem Handgerät 1 das Hautstechmodul 3, das Griffstück 40 mit Griffelementen 41 sowie die hier ein Handstück 2a bildende Antriebsvorrichtung 2 voneinander trennbar oder lösbar. Fig. 11 zeigt die getrennte Anordnung teilweise im Schnitt. Mit dem Gehäuse 4 ist ein Handstück-Gehäuse gebildet.

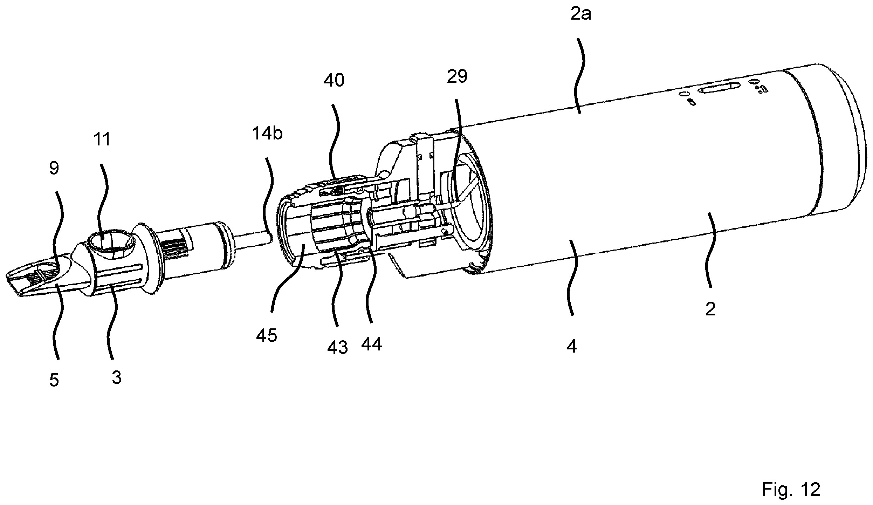

Um bei dem gezeigten Ausführungsbeispiel das Handstück 2a, also das Gehäuse 4 (Handstück-Gehäuse), das Griffstück 40 sowie das Hautstechmodul 3 miteinander funktional zu verbinden, wird zum (zumindest teilweisen) Ausbilden einer ersten mechanischen Verbindung zwischen dem Gehäuse 4 des Handstücks 2a und dem Griffstück 40 zunächst gemäß Fig. 12 das Griffstück 40 mit den Griffelementen 41 auf einen vorderen Gehäuseabschnitt 42 am (Handstück-)Gehäuse 4 aufgesteckt. Hierzu weist das Griffstück 40 sich nach hinten erstreckende, federnde Rastelemente 43 auf, die im gezeigten Beispiel mit Rasthaken gebildet und innenseitig umlaufend angeordnet sind. Die Rastelemente 43 verbinden sich formschlüssig mit einer umlaufenden Elementaufnahme 44 auf der Innenseite des Gehäuseabschnitts 42. In dieser Anordnung ist das Griffstück 40 (nach wie vor) von dem Gehäuse 4 und somit vom Handstück 2a (und somit der Antriebsvorrichtung 2) lösbar, indem das Griffstück 40 (wieder) abgenommen wird.

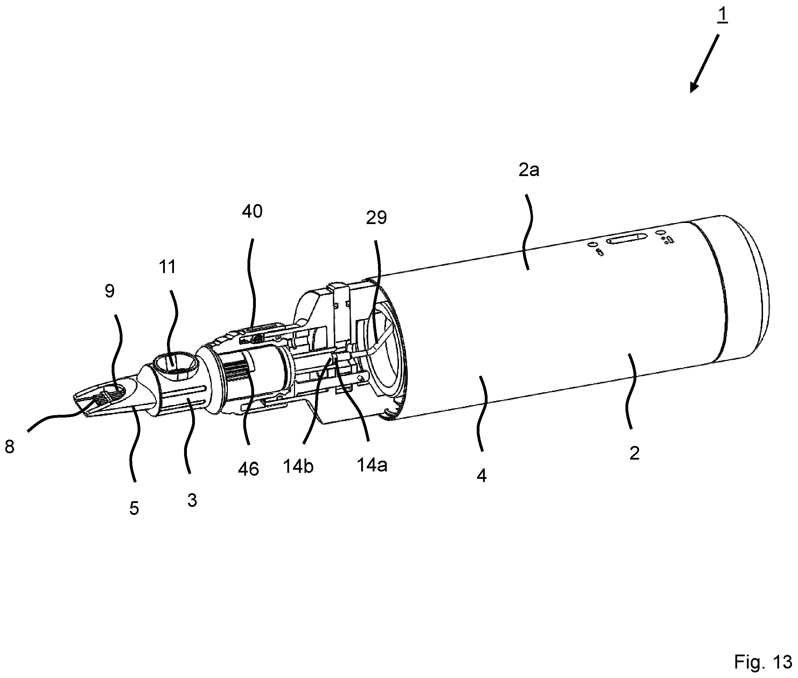

Sodann wird gemäß Fig. 13 das Hautstechmodul 3 in eine Aufnahme 45 des Griffstücks 40 eingesteckt und dort mittels einer Rasteinrichtung 46 verrastet, sodass eine drehfeste Verbindung zwischen Hautstechmodul 3 und Griffstück 40 ausgebildet wird. Hierdurch ist eine zweite mechanische Verbindung zwischen Hautstechmodul 3 und Griffstück 40 hergestellt. Das Ausbilden dieser zweiten mechanischen Verbindung führt dazu, dass nun das Griffstück 40 an dem vorderen Gehäuseabschnitt 42 des (Handstück-)Gehäuses 4 gesichert ist, da die Rastelemente 43 durch das eingesteckte Hautstechmodul 3 gesichert sind, derart, dass die Rastelemente 43 sich nicht mehr aus der Verbindung mit dem innenseitigen Elementaufnahme 44 lösen können. Hierdurch ist die erste mechanische Verbindung zwischen dem Gehäuse 4 des Handstücks 2a und dem Griffstück 40 dann gesichert ausgeführt.

Griffstück 40 und hiermit drehfest verbundenes Hautstechmodul 3 sind dann drehbar mit dem (Handstück-)Gehäuse 4 verbunden, derart, dass das Modulgehäuse 5 (zusammen mit dem Griffstück 40) in unterschiedliche Drehstellungen relativ zum Handstück 2a (oder Antriebsvorrichtung 2) verdreht oder verlagert werden kann. Im Betrieb kann der Nutzer so das Gehäuse 4, also das Handstück 2a, mit der Hand bzw. den Fingern fest greifen und je nach gewünschter Anpassung an unterschiedliche Anwendungsszenarien das Modulgehäuse 5 mit dem Griffstück 40 relativ hierzu drehen, so dass auch die Relativlage der vorderseitigen Öffnung 9 in Bezug auf das Handstück 2a geändert wird.

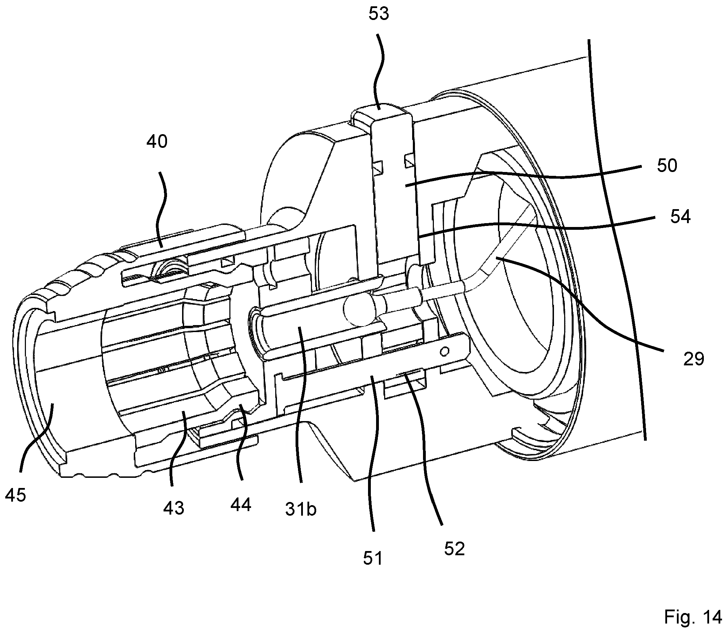

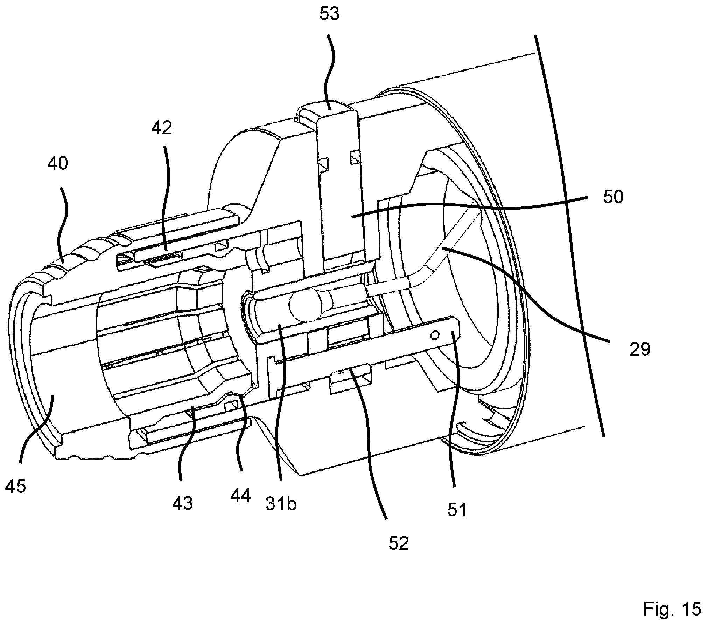

Fig. 14 und 15 zeigen eine Detaildarstellung mit aufgesetztem Griffstück 40 (vergleichbar Fig. 12). Es ist eine Einstelleinrichtung 50 zum Einstellen eines Nadelheraus- oder Nadel- überstands (NHS) gezeigt. Diese weist bei der gezeigten Ausführungsform einen in Längsrichtung verlaufenden Bolzen 51 auf, welcher hier beispielhaft T-förmig ausgeführt ist und auf seiner Oberfläche eine Verzahnung 52 aufweist, die mit einem Sicherungsbauteil 53 im Eingriff steht, welcher vorgespannt ist. Zum Verändern des Nadelherausstands kann das Sicherungsbauteil 53 in einer Bauteilaufnahme 54 manuell nach innen (in Fig. 14 und 15 nach unten) gedrückt werden (gegen eine Federkraft), so dass der Bolzen 51 freigegeben ist für ein Verschieben relativ zum Sicherungsbauteil 53 in Arbeitsrichtung. Fig. 14 und 15 zeigen unterschiedliche Stellungen des Bolzens 51 in Bezug auf das Sicherungsbauteil 53 und somit unterschiedliche Einstellungen für den Nadelherausstand. Dieser verändert sich, indem das Griffstück 40 zusammen mit dem hierin aufgenommen Hautstechmodul 3 in seiner Relativposition zur Verbindungsstelle 29 entlang der Arbeitsrichtung vor- oder zurück verschoben wird, wodurch sich (bei gleichbleibender Hublänge) der Heraus- oder Überstand der Nadelspitze 8 in Bezug auf die Modulgehäuseöffnung 9 verändert.

Es kann vorgesehen sein (nicht dargestellt), den Bolzen 51 mit einem Gewinde zu versehen und hierauf ein Stirnzahnrad mit passendem Innengewinde anzuordnen, welches axial fixiert drehbar gelagert ist und dessen Zähne mit einer passenden Schnecke im Eingriff sind, die von einem Kleinstmotor, zum Beispiel einem 2-Pol-4-Draht-Schrittmotor angetrieben wird, so dass ein Spindeltrieb-Schneckengetriebe ausgebildet ist. Daran kann eine Messmöglichkeit vorgesehen sein, um ein NHS-proportionales elektronisches Signal an die Elektronik des Handgeräts zu übermitteln und den NHS gezielt elektromotorisch einstellen / anzeigen zu können.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Handgerät (1) zum lokalen Aufstechen einer menschlichen oder einer tierischen Haut, aufweisend:
- ein Handstück (2a) mit einem Handstück-Gehäuse (4);
- ein Hautstechmodul (3), welches mit dem Handstück (2a) verbunden ist und ein Modulgehäuse (5) aufweist, an dem eine Modulgehäuseöffnung (9) gebildet ist;
- eine Antriebvorrichtung (2), die zumindest teilweise in dem Handstück-Gehäuse (4) angeordnet und eingerichtet ist, an einer handstückseitigen Verbindungsstelle (14a) entlang einer Arbeitsrichtung eine Vor- und Zurück-Antriebsbewegung mit einer Hublänge bereitzustellen; und
- eine Hautstecheinrichtung (7), die zumindest teilweise in dem Modulgehäuse (5) angeordnet ist und wenigstens eine Stechnadel (6) an einer Nadelaufnahme sowie eine modulseitige Verbindungsstelle (14b) aufweist, welche mit der handstückseitigen Verbindungsstelle (14a) verbunden ist;
wobei
- die Vor- und Zurück-Antriebsbewegung über die modulseitige Verbindungsstelle (14b) auf die Nadelaufnahme übertragbar ist, derart, dass im Betrieb die wenigstens eine Stechnadel (6) zwischen einer hinteren Stellung und einer vorderen Stellung verlagerbar ist, in welcher eine Nadelspitze (8) der wenigstens einen Stechnadel (6) gegenüber der Modulgehäuseöffnung (9) im Umfang eines Nadelherausstands übersteht; und
- das Modulgehäuse (5) mit der Modulgehäuseöffnung (9) relativ zum Handstück-Gehäuse (4) zwischen einer ersten und einer zweiten Drehstellung drehbar ist und ein erster Nadelherausstand in der ersten Drehstellung des Modulgehäuses (5) gleich einem zweiten Nadelherausstand in der zweiten Drehstellung des Modulgehäuses ist.

2. Handgerät (1) nach Anspruch 1, **dadurch gekennzeichnet, dass** das Hautstechmodul (3) mit dem Handstück (2a) lösbar verbunden ist.

3. Handgerät (1) nach Anspruch 2, **dadurch gekennzeichnet, dass** das Hautstechmodul (3) in Bezug auf das Handstück (2a) zwischen einer gekoppelten Stellung, in welcher das Hautstechmodul (3) mit dem Handstück (2a) funktional verbunden ist, und einer entkoppelten Stellung verlagerbar ist, in welcher das Hautstechmodul (3) von dem Handstück (2a) getrennt ist.

4. Handgerät (1) nach mindestens einem der vorangehenden Ansprüche, **dadurch gekennzeichnet, dass** das Modulgehäuse (5) mit der Modulgehäuseöffnung (9) relativ zum Handstück-Gehäuse (4) zwischen der ersten und der zweiten Drehstellung stufenlos in weitere Drehstellungen drehbar ist, in denen der Nadelherausstand jeweils gleich dem ersten Nadelherausstand ist.

5. Handgerät (1) nach mindestens einem der vorangehenden Ansprüche, **dadurch gekennzeichnet, dass** der Nadelherausstand mittels einer Einstelleinrichtung (50) veränderbar ist.

6. Handgerät (1) nach Anspruch 5, **dadurch gekennzeichnet, dass** für mehrere verschieden eingestellte Nadelherausstände jeweils der erste Nadelherausstand in der ersten Drehstellung des Modulgehäuses (5) gleich dem zweiten Nadelherausstand in der zweiten Drehstellung des Modulgehäuses (5) ist, wenn das Modulgehäuse (5) mit der Modulgehäuseöffnung (9) relativ zum Handstück-Gehäuse (4) zwischen der ersten und der zweiten Drehstellung verlagert wird.

7. Handgerät (1) nach mindestens einem der vorangehenden Ansprüche, **gekenn- zeichnet** durch ein Griffstück (40), welches mit dem Hautstechmodul (3) drehfest und dem Handstück (2a) verdrehbar verbunden ist, wenn das Handstück (2a) und das Hautstechmodul (3) verbunden sind.

8. Handgerät (1) nach Anspruch 7, **dadurch gekennzeichnet, dass** das Griffstück (40) mittels einer ersten mechanischen Verbindung mit dem Handstück (2a) verbunden ist.

9. Handgerät (1) nach Anspruch 7 oder 8, **dadurch gekennzeichnet, dass** das Griffstück (40) mittels einer zweiten mechanischen Verbindung mit dem Hautstechmodul (3) verbunden ist.

10. Handgerät (1) nach mindestens einem der Ansprüche 7 bis 9, dadurch **gekenn- zeichnet**, dass das Griffstück (40) mit dem Hautstechmodul (3) und / oder dem Handstück (2a) lösbar verbunden ist.

11. Handgerät (1) nach Anspruch 10, **dadurch gekennzeichnet, dass** die erste mechanische Verbindung zwischen dem Griffstück (40) und dem Handstück (2a) gegen ein Trennen der ersten mechanischen Verbindung gesichert ist, wenn das Griffstück (40) und das Hautstechmodul (3) mittels der zweiten mechanischen Verbindung verbunden sind.

12. Handgerät (1) nach mindestens einem der Ansprüche 7 bis 11, dadurch **gekenn- zeichnet**, dass das Griffstück (40) mit einer auf einen Gehäuseabschnitt (42) des Handstück-Gehäuse (4) aufsteckbaren Griffhülse gebildet ist.

13. Handgerät (1) nach mindestens einem der vorangehenden Ansprüche, **dadurch gekennzeichnet, dass** die Modulgehäuseöffnung (9) eine Öffnungsfläche aufweist, die in der ersten Drehstellung eine erste Relativlage zum Handstück-Gehäuse (4) und in der zweiten Drehstellung eine zweite Relativlage zum Handstück-Gehäuse (4) aufweist, welche von der ersten Relativlage verschieden ist.

14. Handgerät (1) nach mindestens einem der vorangehenden Ansprüche, **dadurch gekennzeichnet, dass** die Hublänge mittels einer Hubeinstelleinrichtung (25) veränderbar ist.

15. Handgerät (1) nach Anspruch 14, **dadurch gekennzeichnet, dass** die Hubeinstelleinrichtung (25) eingerichtet ist, zum Einstellen der Hublänge eine Relativlage zwischen der Nadelaufnahme mit der wenigstens einen Stechnadel (6) und der Modulgehäuseöffnung (9) entlang der Arbeitsrichtung einzustellen.

## Claims

1. Hand-held device (1) for locally puncturing a human or an animal skin, comprising:
- a handpiece (2a) with a handpiece housing (4);
- a skin puncturing module (3) which is connected to the handpiece (2a) and has a module housing (5) on which a module housing opening (9) is formed;
- a drive device (2) which is arranged at least partially in the handpiece housing (4) and is configured to provide a forward and backward drive movement with a stroke length along a working direction at a handpiece-side connection point (14a); and
- a skin puncturing device (7) which is arranged at least partially in the module housing (5) and comprises at least one puncturing needle (6) on a needle receptacle and a module-side connection point (14b) which is connected to the handpiece-side connection point (14a);
wherein
- the forward and backward drive movement can be transmitted to the needle receptacle via the module-side connection point (14b) in such a way that, during operation, the at least one puncturing needle (6) can be displaced between a rear position and a front position in which a needle tip (8) of the at least one puncturing needle (6) protrudes with respect to the module housing opening (9) to the extent of a needle protrusion; and
- the module housing (5) with the module housing opening (9) can be rotated relative to the handpiece housing (4) between a first and a second rotational position and a first needle protrusion in the first rotational position of the module housing (5) is equal to a second needle protrusion in the second rotational position of the module housing.

2. Hand-held device (1) according to Claim 1, **characterized in that** the skin puncturing module (3) is releasably connected to the handpiece (2a).

3. Hand-held device (1) according to Claim 2, **characterized in that** the skin puncturing module (3) can be displaced with respect to the handpiece (2a) between a coupled position, in which the skin puncturing module (3) is functionally connected to the handpiece (2a), and a decoupled position, in which the skin puncturing module (3) is separated from the handpiece (2a).

4. Hand-held device (1) according to at least one of the preceding claims, **characterized in that** the module housing (5) with the module housing opening (9) can be rotated continuously relative to the handpiece housing (4) between the first and the second rotational position into further rotational positions, in which the needle protrusion is in each case equal to the first needle protrusion.

5. Hand-held device (1) according to at least one of the preceding claims, **characterized in that** the needle protrusion can be varied by means of an adjusting device (50).

6. Hand-held device (1) according to Claim 5, **characterized in that**, for a plurality of differently adjusted needle protrusions, in each case the first needle protrusion in the first rotational position of the module housing (5) is equal to the second needle protrusion in the second rotational position of the module housing (5) when the module housing (5) with the module housing opening (9) is displaced relative to the handpiece housing (4) between the first and the second rotational position.

7. Hand-held device (1) according to at least one of the preceding claims, **characterized by** a handle piece (40) which is connected to the skin puncturing module (3) in a rotationally fixed manner and to the handpiece (2a) in a rotatable manner when the handpiece (2a) and the skin puncturing module (3) are connected.

8. Hand-held device (1) according to Claim 7, **characterized in that** the handle piece (40) is connected to the handpiece (2a) by means of a first mechanical connection.

9. Hand-held device (1) according to Claim 7 or 8, **characterized in that** the handle piece (40) is connected to the skin puncturing module (3) by means of a second mechanical connection.

10. Hand-held device (1) according to at least one of Claims 7 to 9, **characterized in that** the handle piece (40) is releasably connected to the skin puncturing module (3) and/or the handpiece (2a).

11. Hand-held device (1) according to Claim 10, **characterized in that** the first mechanical connection between the handle piece (40) and the handpiece (2a) is secured against separation of the first mechanical connection when the handle piece (40) and the skin puncturing module (3) are connected by means of the second mechanical connection.

12. Hand-held device (1) according to at least one of Claims 7 to 11, **characterized in that** the handle piece (40) is formed with a handle sleeve which can be fitted onto a housing section (42) of the handpiece housing (4).

13. Hand-held device (1) according to at least one of the preceding claims, **characterized in that** the module housing opening (9) has an opening surface which, in the first rotational position, has a first relative position with respect to the handpiece housing (4) and, in the second rotational position, has a second relative position with respect to the handpiece housing (4), which second relative position is different from the first relative position.

14. Hand-held device (1) according to at least one of the preceding claims, **characterized in that** the stroke length can be varied by means of a stroke adjusting device (25).

15. Hand-held device (1) according to Claim 14, **characterized in that** the stroke adjusting device (25) is configured to adjust a relative position between the needle receptacle with the at least one puncturing needle (6) and the module housing opening (9) along the working direction in order to adjust the stroke length.

## Revendications

1. Appareil à main (1) permettant de piquer localement une peau humaine ou animale, présentant :
- une pièce à main (2a) dotée d'un boîtier de pièce à main (4) ;
- un module de piquage de peau (3) qui est relié à la pièce à main (2a) et présente un boîtier de module (5) sur lequel est pratiquée une ouverture de boîtier de module (9) ;
- un dispositif d'entraînement (2) qui est disposé au moins partiellement dans le boîtier de pièce à main (4) et conçu pour exercer un mouvement d'entraînement vers l'avant et vers l'arrière avec une longueur de course à un point de liaison côté pièce à main (14a) le long d'un sens de travail ; et
- un dispositif de piquage de peau (7) qui est disposé au moins partiellement dans le boîtier de module (5) et présente au moins une aiguille de piquage (6) au niveau d'un support d'aiguille et un point de liaison situé côté module (14b) et relié au point de liaison côté pièce à main (14a) ;
- le mouvement d'entraînement vers l'avant et vers l'arrière étant transmissible, par l'intermédiaire du point de liaison situé côté module (14b), au support d'aiguille de manière à ce que, en cours de fonctionnement, une aiguille de piquage (6) soit déplaçable entre une position arrière et une position avant dans laquelle une pointe d'aiguille (8) de l'au moins une aiguille de piquage (6) dépasse par rapport à l'ouverture de boîtier de module (9) à raison d'une protubérance de l'aiguille ; et
- le module de boîtier (5) pouvant tourner avec l'ouverture de boîtier de module (9) par rapport au boîtier de pièce à main (4) entre une première une seconde position de rotation et une première protubérance de l'aiguille étant, dans la première position de rotation du boîtier de module (5), égale à une seconde protubérance de l'aiguille dans la seconde position de rotation du boîtier de module.

2. Appareil à main (1) selon la revendication 1, **caractérisé en ce que** le module de piquage de peau (3) est relié de manière dissociable à la pièce à main (2a).

3. Appareil à main (1) selon la revendication 2, **caractérisé en ce que** le module de piquage de peau (3) est relié fonctionnellement par rapport à la pièce à main (2a) entre une position accouplée dans laquelle le module de piquage de peau (3) est relié fonctionnellement à la pièce à main (2a), et une position désaccouplée dans laquelle le module de piquage de peau (3) est séparé de la pièce à main (2a).

4. Appareil à main (1) selon au moins une des revendications précédentes, **caractérisé en ce que** le boîtier de module (5) peut tourner avec l'ouverture de boîtier de module (9) par rapport au boîtier de pièce à main (4) entre la première et la seconde position de rotation progressivement dans d'autres positions de rotation dans lesquelles la protubérance de l'aiguille est respectivement égale à la première protubérance de l'aiguille.

5. Appareil à main (1) selon au moins une des revendications précédentes, **caractérisé en ce que** la protubérance de l'aiguille est modifiable au moyen d'un dispositif de réglage (50).

6. Appareil à main (1) selon la revendication 5, **caractérisé en ce que**, pour plusieurs protubérances d'aiguille réglées différemment, la première protubérance de l'aiguille dans la première position de rotation du boîtier de module (5) est respectivement égale à la seconde protubérance de l'aiguille dans la seconde position de rotation du boîtier de module (5) lorsque le boîtier de module (5) est déplacé avec l'ouverture de boîtier de module (9) par rapport au boîtier de pièce à main (4) entre la première et la seconde position de rotation.

7. Appareil à main (1) selon au moins une des revendications précédentes, **caractérisé par** une poignée (40) qui est reliée de manière fixe en rotation au module de piquage de peau (3) et de manière à pouvoir tourner à la pièce à main (2a) lorsque la pièce à main (2a) et le module de piquage de peau (3) sont raccordés.

8. Appareil à main (1) selon la revendication 7, **caractérisé en ce que** la poignée (40) est reliée au moyen d'une première connexion mécanique à la pièce à main (2a).

9. Appareil à main (1) selon la revendication 7 ou 8, **caractérisé en ce que** la poignée (40) est reliée au moyen d'une seconde connexion mécanique au module de piquage de peau (3).

10. Appareil à main (1) selon au moins une des revendications 7 à 9, **caractérisé en ce que** la poignée (40) est reliée de manière dissociable au module de piquage de peau (3) et/ou à la pièce à main (2a).

11. Appareil à main (1) selon la revendication 10, **caractérisé en ce que** la première connexion mécanique entre la poignée (40) et la pièce à main (2a) est bloquée pour éviter une séparation de la première connexion mécanique lorsque la poignée (40) et le module de piquage de peau (3) sont reliés au moyen de la connexion mécanique.

12. Appareil à main (1) selon au moins une des revendications 7 à 11, **caractérisé en ce que** la poignée (40) est conformée avec un manchon de préhension pouvant être fiché sur une section de boîtier (42) du boîtier de pièce à main (4).

13. Appareil à main (1) selon au moins une des revendications précédentes, **caractérisé en ce que** l'ouverture de boîtier de module (9) présente une surface d'ouverture qui présente, dans la première position de rotation, une première position relative par rapport au boîtier de pièce à main (4) et, dans la seconde position de rotation, une seconde position relative par rapport au boîtier de pièce à main (4), laquelle seconde position est différente de la première position relative.

14. Appareil à main (1) selon au moins une des revendications précédentes, **caractérisé en ce que** la longueur de course d'au moins un dispositif de réglage de course (25) est modifiable.

15. Appareil à main (1) selon la revendication 14, **caractérisé en ce que** le dispositif de réglage de course (25) est conçu pour régler la longueur de course dans une position relative entre le support d'aiguille comportant l'au moins une aiguille de piquage (6) et l'ouverture de boîtier de module (9) le long du sens de travail.
